# DEMANDE DE BREVET EUROPEEN

(11) **EP 0 630 653 A1**
(43) Date de publication de la demande: **28.12.1994**
(21) Numéro de dépôt: 94440038.1
(22) Date de dépôt: 08.06.1994
(51) Int. Cl.: A61L 27/00

(54) **Composition destinée à favoriser la formation et le développement des cellules osseuses**

(30) Priorité: 08.06.1993 FR 9306820
(71) Demandeur: AMP DEVELOPPEMENT, F-69100 Villeurbanne (FR); TEXINFINE, F-69006 Lyon (FR)
(72) Inventeur: Augagneur, Christian, 69007 Lyon (FR); Barbié, Christian, 69009 Lyon (FR); Gutierrez, Gilles, 69006 Lyon (FR); Serrar, Mostafa, 69100 Villeurbanne (FR)
(74) Mandataire: Polus, Camille

(57) **Abrégé**

Composition pour favoriser la formation et le développement des cellules osseuses.

Elle comporte de l'acide désoxyribonucléique (ADN) sur lequel sont fixés des vecteurs actifs modulant l'expression phénotypique des cellules osseuses, ainsi qu'une quantité physiologiquement acceptable d'un stabilisant du pH.

Les vecteurs modulants actifs sont choisis parmi les tocophérols, les caroténoïdes et les xanthophyles.

## Description

La présente invention se rapporte à une composition pour stimuler la formation et le développement des cellules osseuses.

La stimulation de la formation et du développement des cellules osseuses s'avère nécessaire à chaque fois qu'interviennent des phénomènes de consolidations ou de repousses osseuses. Il s'agit par exemple de la consolidation des fractures osseuses qu'elles soient d'origine accidentelle ou chirurgicale (ostéotomies, ostéoclasies, etc.), de la reconstruction ou reconstitution des tissus osseux, ou bien de la fixation biologique des prothèses articulaires ou d'implants osseux. Il peut également s'agir de cas pathologiques, dans lesquels l'os repousse mal ou ne repousse pas, ou les fractures consolident mal ou pas (perte de substance osseuse, pseudarthrose, etc.)

Selon l'état actuel de la technique, les chirurgiens utilisent divers types de substituts osseux ou matériaux de comblement, qu'ils soient naturels (corail, etc) ou synthétiques, pour obturer certaines cavités ou remplacer certains défects osseux. Or, l'usage de telles substances hétérogènes présente l'inconvénient de modifier l'expression phénotypique des ostéocytes en contact avec elles et elles n'ont pas toujours fait la preuve de leur capacité à favoriser la formation de l'os. D'autre part, les implants dits à fixation biologique présentent à la surface de contact avec l'os une structure poreuse, dans les pores de laquelle le tissu osseux pénètre pour assurer la fixation de l'implant à l'os. Selon la structure poreuse en présence, la repousse osseuse est très imparfaite, entraînant une mauvaise fixation de l'implant.

Le but de la présente invention consiste donc à fournir un matériau de comblement osseux qui ne présente pas les inconvénients précités et surtout qui favorise la formation et le développement des cellules osseuses (stimulation de l'ostéogénèse) assurant le comblement de la cavité ou du défect osseux et favorisant la repousse osseuse et l'ostéointégration des implants de quelque forme ou structure par du tissu osseux.

La composition de l'invention, qui vise à atteindre le but précité,comporte de l'acide désoxyribonucléique sur lequel sont fixés des vecteurs actifs modulant l'expression phénotypique des cellules osseuses, ainsi qu'une quantité physiologiquement acceptable d'un stabilisant du pH.

L'acide désoxyribonucléique (ADN) utilisé dans la composition selon l'invention peut être d'origine végétale ou animale. Toutefois, il est préférable d'éviter les ADN extraits de mammifères, ceci afin de garantir à l'être humain sur lequel cette composition va être appliquée l'absence de contaminants pathogènes transmissibles. A titre d'exemple préférentiel, on citera l'ADN de saumon.

Les vecteurs fixés sur l'ADN sont destinés à moduler l'activité des cellules et à "dé-stresser" celles-ci pour leur redonner leur capacité de production et leur vitalité physiologique. Cette modulation permettra aux cellules concernées de croître, de migrer, et de réaliser les synthèses nécessaires à l'établissement de nouvelles colonies osseuses. On peut citer comme additifs à la trame polymérisée (ADN) les xanthophyles tels que l'astaxanthine ou la canthaxanthine, ainsi que les caroténoïdes tels que les lycopènes, les α et β carotènes, ou encore les tocophérols sous leurs différentes formes isomériques.

De plus, la composition selon l'invention peut contenir au moins un stabilisant du pH destiné à maintenir une valeur physiologique de celui-ci (7, 35 ± 0,15), par exemple un sel de l'acide phosphorique comme un phospate acide de sodium, de potassium ou d'ammonium, et de préférence le phosphate acide monosodique NaH₂PO₄, lequel présente en outre l'intérêt de contribuer à la consolidation osseuse par apport de phosphore.

Cette composition peut également contenir des adjuvants de structure, tels que les polypeptides (albumine, collagène, etc.), les polyosides (diholosides comme la saccharose, polyholosides comme la cellulose, dextrans, chitine ou chitosan, etc.), ainsi que d'autres éléments tels que des agents conservateurs et des antioxydants. Il peut s'agir par exemple de tocophérol (acétate), de palmitate d'acide ascorbique ou d'hydroxyproline, ou encore de lécithine à très bas indice d'iode, notamment le di-palmitate et le distéarate de phosphatidylcholine, et de glycérol. Ces composés présentent généralement une bonne inertie à la radiostérilisation à laquelle la composition selon l'invention est en principe soumise.

Enfin, le nouveau matériau selon l'invention peut se présenter, comme cela sera décrit plus en détails ci-après, sous plusieurs formes différentes selon l'utilisation prévue, par exemple sous forme de gel concentré, sous forme lyophilisée, sous forme de poudre, etc.

D'une manière générale, le procédé de fabrication de la composition selon l'invention comprend le broyage des fibres d'ADN choisies, éventuellement par cryobroyage en présence d'azote liquide, la fixation des vecteurs modulants actifs et l'adjonction d'une quantité appropriée de stabilisant du pH, par exemple du NaH₂PO₄, de telle sorte que le pH d'une solution d'ADN dans l'eau soit au niveau physiologique et enfin l'adjonction éventuelle d'autres adjuvants tels que des agents facilitant la lyophilisation ou la dissolution. Le produit est ensuite séché sous vide, puis stérilisé de préférence par radiostérilisation, et enfin conditionné sous forme d'un produit utilisable et commercialisable.

Plus particulièrement, on peut utiliser comme produit de base de la composition un ADN commercial, par exemple de la laitance de saumon, qui est généralement salifié et présente ainsi un pH trop élevé, qu'il sera nécessaire d'abaisser au niveau physiologique, par exemple avec un phosphate acide de sodium, de potassium ou d'ammonium. La quantité d'agent stabilisant du pH à utiliser dépend notamment de la provenance de l'ADN, et varie généralement entre environ 0,1 et 10 % de la composition.

Afin que le gel ait une tenue sur l'os, la concentration en ADN pourra être assez élevée, en pratique entre environ 1 et 25 %. Toutefois, si les concentrations en ADN supérieures à 15 % présentent un intérêt plastique, elles sont difficiles à employer lors d'une intervention et nécessitent le recours à une pompe ou autre dispositif approprié pour extruder le gel à partir de son conditionnement.

Le gel peut également être stérilisé à chaud, mais dans ce cas les conditions de cette stérilisation doivent respecter le fait que l'ADN ne doit pas être remué lors des refroidissements, et que le gel ne se reconstitue plus au-delà d'une certaine température, ni après un certain nombre de cycles de chauffage et de refroidissement.

On peut également prévoir pour remédier à cet inconvénient un gel extemporané à partir d'une poudre radiostérilisée et d'eau stérilisée. Un conditionnement à deux compartiments est utilisable pour permettre le mélange eau-poudre sans nécessité d'ouvrir ledit conditionnement. Dans le cas de la poudre prêté à gélifier, la composition contient en plus des agents facilitant la dissolution. Il peut s'agir de sucres, d'acides aminés, de peptides ou de sels.

A titre d'exemple, on peut citer des sels tels que le chlorure de sodium ou de potassium, des sels d'acétates alcalins, des phosphates, des sulfates, des sels d'acides osidiques, ou des dérivés d'oses tels que l'acide glucuronique, le glucophosphate lactogluconique, etc. Les oses utilisables pour faciliter la dissolution sont choisis de préférence parmi les oses simples, les diholosides (saccharose, lactose, thréalose, etc.), les polyholosides commes les dextrans, etc.

L'application de gel sur un site opératoire peut présenter des inconvénients ou du moins des difficultés de mise en oeuvre. Pour y remédier, il est possible de prévoir un gel lyophilisé, dont la consistance et la tenue lui confère des propriétés particulières, le matériau prenant l'aspect d'un tissu non tissé. En effet, la tenue du matériau est assez souple pour la mettre en forme ; puis, une fois humidifiée, la plaque lyophilisée reprend sa consistance de gel et conserve ses propriétés pharmacodynamiques vis-à-vis des cellules. Des lyophilisats obtenus à partir de solutions concentrées permettent d'obtenir des matériaux très rigides aptes à combler des cavités osseuses. De telles formulations de lyophilisats comportent des adjuvants de lyophilisation qui doivent bien entendu répondre notamment aux exigences suivantes absence de cytotoxicité, possibilité d'être admis comme produits pharmaceutiques, être radiostérilisables, c'est-à-dire être susceptibles de garder leur propriétés après stérilisation, ne pas altérer les propriétés de la composition et ne pas modifier les propriétés galéniques du produit.

En général, ces adjuvants de lyophilisation sont choisis préférentiellement parmi les sucres, dont les oses simples et les polyoses, les sels, les sels minéraux, certains peptides et les acides aminés, par exemple le thréalose.

Au gel d'ADN, qu'il soit issu d'un lyophilisat, d'une poudre à gélification instantanée ou d'un gel préconstitué, on ajoute selon l'invention des adjuvants actifs qui confèrent à la composition des propriétés spécifiques, par exemple un effet antistress cellulaire et une modulation de l'expression phénotypique vers la synthèse du procollagène III.

La plupart des caroténoïdes permettent de développer des effets antistress sur les cellules (le stress cellulaire étant la perte de la capacité à migrer et à se diviser), par exemple le β-carotène, l'astaxantine, la canthaxantine, le lycopène, etc.

Le β-carotène, lorsqu'il est encapsulé dans une structure vésiculaire, présente donc un effet antistress, c'est-à-dire qu'il permet de restaurer l'aspect et le fonctionnement des cellules quand elles sont soumises à un stress physique (thermique ou mécanique), biologique ou chimique (par des stéroïdes). La concentration utilisée varie de 0,001 à 1 % environ de la solution vésiculaire qui peut contenir à titre purement indicatif 6 % de phospholipides, 0,5 % de cholestérol, 0,05 % de tocophérol, 0,05 % d'ascorbate de sodium et 0,05 % de mannitol. La taille des vecteurs est de l'ordre de 200 nm ± 50, et leur potentiel zéta est d'environ - 30mV (cette valeur pouvant toutefois considérablement varier selon l'épaisseur de la couche de Stern). Les phospholipides utilisés doivent être radiostérilisables et sont généralement des acides gras à degré de saturation élevé (indice d'iode faible).

La modulation phénotypique des cellules mésenchymateuses pour produire du collagène III (collagène cicatriciel) est obtenue grâce à des vecteurs dont la structure et la composition orientent la synthèse cellulaire vers ce collagène III de manière préférentielle (AMPC III^{R}).

Les vecteurs actifs sont généralement de structure nanovésiculaire, soit de nature liposomiale ou capsulaire.

Les liposomes sont les vecteurs les plus aisés à mettre en oeuvre. Les vecteurs vésiculaires du genre nanocapsulaire sont également utilisables, tels que ceux obtenus selon les méthodes connues décrites par Leverge (polymère d'acide polylactique glycolique) ou par Couvreur (polymère de cyanoacrylate).

D'autre part, on peut considérer trois types de transporteurs actifs :
a) les vecteurs de taille perceptible à l'oeil nu, qui sont généralement dénommés sphères, sphérules, perles, microperles, etc ;
b) ceux dont la taille est comprise entre 1 et 600 µm, qui sont les microvecteurs, qui peuvent être déplacés grâce à une force mécanique ou un champ électrique, et qui se subdivisent en vecteurs pleins (microsphères), vecteurs creux (microcapsules) et vecteurs de nature spongieuse (microsponges) ;
c) les vecteurs de taille nanométrique, qui ont une vitesse de déplacement fonction de la température et qui présentent une bonne affinité cellulaire ; on distingue les vecteurs pleins ou nanosphères, les vecteurs creux ou nanovésicules (si la limitante entre le milieu interne du vecteur et l'extérieur est constituée par une membrane formée à base de phospholipides on parle de liposomes, s'il s'agit d'une paroi formée par un milieu homogène, on parle de nanocapsules, et si elle a une structure en maille ou en grillage, on utilise le terme de rétoïdes), et les vecteurs de nature spongieuse (structures matricielles dans lesquelles circulent des canalicules ou des vésicules forment une structure ouverte ou fermée).

A titre d'exemple, on peut citer les formulations suivantes :
(1) Gel préconstitué (pour 100 g)
   - ADN: 10 g
   - Liposomes de β-carotène: 1 g
   - Liposomes AMPC III^{R}: 1 g
   - NaH₂PO₄: 3,26 g
   - Eau: QS
(2) Poudre à gélification instantanée (pour un gel de 100ml)
   - ADN: 10 g
   - Liposomes de β-carotène: 1 g
   - Liposomes AMPC III^{R}: 1 g
   - NaH₂PO₄: 3,26 g
   - Thréalose: 1,5 g
   - NaCl: 0,25 g
   - Dextrant 50000: 1g
(3) Matériau souple obtenu par lyophilisation (pour 100ml)
   - ADN: 1 g
   - Collagène: 1 g
   - NaH₂PO₄: QS pH 7
   - Thréalose: 0,2 g
   - Liposomes de β-Carotène: 0,01 g
   - Liposomes AMPC III^{R}: 0,01 g
   - Eau: QS
(4) Matériau dur obtenu par lyophilisation (pour 100 ml)
   - ADN: 5 g
   - Dextran 60 000: 0,1 g
   - NaH₂PO₄: QS pH 7
   - Thréalose: 1 g
   - Liposomes de β carotène: 0,05 g
   - Liposomes AMPC III^{R}: 0,05 g
   - Eau: QS

Les deux produits (3) et (4) peuvent être coulés sous forme de plaques d'épaisseur et de forme variables. Ces produits solides peuvent donc être de consistance variable, allant de très souple (type coton hydrophile) à dur (type bois), avec toutes les gradations intermédiaires.

Les compositions selon l'invention peuvent être avantageusement utilisées non seulement comme matériau de comblement osseux, mais également pour toutes les applications mentionnées à titre d'exemples dans l'introduction de la présente description.

On a pu constater en pratique que lorsqu'une composition selon l'invention est appliquée par exemple dans une cavité osseuse formée artificiellement, elle modifie sensiblement le phénomène de la cicatrisation osseuse en l'améliorant. Il convient de rappeler que la synthèse de l'os trabéculaire (os qui transmet les forces) est précédée d'une étape correspondant à l'élaboration d'un tissu précurseur de l'os, l'ostéoïde. Or, l'utilisation de la composition selon l'invention permet de doubler la vitesse de formation de cet ostéoïde.

Lors du processus d'ossification, l'osteoïde disparaît au profit de l'os. Dans le cadre de tests pratiques, on a constaté en effet que la masse osseuse globale était doublée en utilisant la composition selon l'invention par rapport à un produit témoin habituel, ceci sans que l'espace intertrabéculaire, qui permet au processus nutritif de circuler, ne soit modifié.

La troisième étape de l'ossification consiste en l'intégration de l'os néoformé dans l'os ancien (ostéointégration). Ce processus biologique est caractérisé par le remodelage osseux qui se traduit histologiquement par la présence de surface érodée. On a constaté que le matériau selon l'invention provoque une amplification du remaniement osseux (construction des trabécules osseuses) d'un facteur 7.

On peut ainsi conclure que la composition selon l'invention est efficace notamment pour favoriser la cicatrisation osseuse, pour stimuler la formation des ostéoïdes et des trabécules osseux sans modifier les espaces intertrabéculaires, et pour améliorer l'ostéointégration.

## Revendications

**1)** Composition pour favoriser la formation et le développement des cellules osseuses, caractérisée par le fait qu'elle comporte de l'acide désoxyribonucléique (ADN) sur lequel sont fixés des vecteurs actifs modulant l'expression phénotypique des cellules osseuses, ainsi qu'une quantité physiologiquement acceptable d'un stabilisant du pH.

**2)** Composition selon la revendication 1, caractérisée en ce que l'ADN est présent en une quantité de 1 à 25 %.

**3)** Composition selon la revendication 1 ou la revendication 2, caractérisée en ce qu'elle comporte une quantité de stabilisant permettant de maintenir le pH à une valeur physiologique de 7,35 ± 0,15, ce stabilisant du pH étant choisi parmi les phosphates acides de sodium, de potassium et d'ammonium.

**4)** Composition selon la revendication 3, caractérisée en ce qu'elle comporte de 0,1 à 10 % de phosphate acide monosodique.

**5)** Composition selon l'une des revendications 1 à 4, caractérisée en ce que les vecteurs modulants actifs sont choisis parmi les tocophérols, les carotenoïdes tels que les carotènes α et β et les lycopènes, et les xanthophyles tels que l'astaxanthine et la canthaxanthine.

**6)** Composition selon l'une des revendications 1 à 5, caractérisée en ce qu'elle comporte encore d'autres adjuvants choisis parmi les polypetides tels que l'albumine et le collagène, les diholosides tels que le saccharose et le lactose, les polyholosides tels que les dextrans, la cellulose, la chitine et le chitosan, le palmitate d'acide ascorbique, le distéarate de phosphatidylcholine, l'hydroxyproline et le glycérol.

**7)** Composition selon l'une des revendications 1 à 6, caractérisée en ce qu'elle se présente sous la forme d'un gel, d'une poudre à gélification instantanée, d'un gel lyophilisé, d'un lyophilisat, ou d'un solide de consistance variable.

**8)** Composition selon la revendication 7 sous la forme d'une poudre à gélification instantanée par mélange avec de l'eau, caractérisée en ce qu'elle renferme des agents pour améliorer la prise rapide du gel, par exemple choisis parmi les oses tels que les oses simples, les diholosides comme le saccharose et le lactose et les polyholosides comme les dextrans, les acides aminés, les peptides et les sels tels que les acétates alcalins, les phosphates, les sulfates et les sels d'acides osidiques.

**9)** Composition selon la revendication 7 sous la forme d'un lyophilisat, caractérisée en ce qu'elle comporte comme adjuvants de lyophilisation des oses simples, des polyoses, des sels minéraux, des peptides et/ou des acides aminés.

**10)** Procédé pour la préparation d'une composition pour favoriser la formation et le développement des cellules osseuses selon l'une des revendications 1 à 9, caractérisé en ce qu'on broye des fibres d'ADN, on ajoute à la poudre obtenue des vecteurs modulants actifs et d'éventuels adjuvants, on ajoute un stabilisant de pH de telle sorte que la solution aqueuse de la composition présente un pH physiologique, qu'on sèche le mélange en poudre et qu'on stérilise celui-ci, puis qu'on conditionne la composition obtenue.

**11)** Utilisation de la composition selon l'une des revendications 1 à 9 pour stimuler la formation des osteoïdes et des trabécules osseux afin de favoriser la cicatrisation osseuse et l'ostéointégration, et/ou pour favoriser les repousses osseuses et l'ostéointégration d'un implant de quelque forme ou structure dans un tissu osseux.

**12)** Utilisation de la composition selon l'une des revendications 1 à 9 comme matériau de comblement osseux.
